# FASCICULE DE BREVET EUROPEEN

(11) **EP 3 849 329 B1**
(45) Date de publication et mention de la délivrance du brevet: **20.05.2026**
(21) Numéro de dépôt: 19765746.3
(22) Date de dépôt: 13.09.2019
(51) Int. Cl.: A23D 9/04, A61K 36/02, A23L 33/12, A61K 8/9706, A23D 9/02

(54) **PROCEDE D'EXTRACTION D'UNE HUILE RICHE EN ACIDES GRAS POLYUNSATURES (AGPI)**
VERFAHREN ZUR EXTRAKTION EINES ÖLS ENTHALTEND MEHRFACH UNGESÄTTIGTE FETTSÄUREN
PROCESS FOR THE EXTRACTION OF AN OIL COMPRISING POLYUNSATURATED FATTY ACIDS (PUFAS)

(30) Priorité: 14.09.2018 FR 1858293
(43) Date de publication de la demande: 21.07.2021
(73) Titulaire: Fermentalg, 33500 Libourne (FR)
(72) Inventeur: BOURLES, Louis, 33000 BORDEAUX (FR); SEGUELA, Benjamin, 33500 LIBOURNE (FR); CADERBY, Emma, 33150 CENON (FR); GRIFFITHS, Hywel, 33500 LIBOURNE (FR)
(74) Mandataire: Icosa
(86) Numéro de dépôt international: PCT/EP2019/074461
(87) Numéro de publication internationale: WO 2020/053375

(56) Documents cités:
- WO-A1-2018/011275
- FR-A- 1 186 824
- US-A1- 2011 295 028
- US-A1- 2014 350 222
- US-A1- 2015 176 042
- US-A1- 2016 319 218
- US-B2- 6 750 048

## Description

### DOMAINE DE L'INVENTION

La présente invention concerne un procédé d'extraction d'une huile riche en acides gras polyinsaturés (PUFA), notamment d'une huile de microorganismes riche en acide docosahexaénoïque (DHA, C22:6n3), en particulier d'huiles comprenant plus de 60% de PUFA par rapport à la masse totale de matière grasse.

### ETAT DE LA TECHNIQUE

Il existe une aujourd'hui une demande d'huiles concentrées à fortes teneurs en PUFA, soit pour la fourniture de produits concentrés, comme des gélules d'huile concentrée qui permettent de diminuer les prises pour une quantité équivalent de PUFA. Pour obtenir des huiles à forte teneur en PUFA (par exemple supérieur à 55% de DHA), les huiles sont concentrées par un procédé qui transforme les triglycérides en ethyl esters en impliquant l'utilisation de solvant tel que l'éthanol. Les ethyl esters sont une forme chimique artificielle, ils ne sont pas présents dans la nature. La biodisponibilité des acides gras sous forme d'ethyl esters est bien moindre que sous forme de triglycérides (Ghasemifard et al., 2014). De plus, le procédé élimine les vitamines et les anti-oxidants qui sont présents dans l'huile brute. Par conséquent, l'huile concentrée est plus vulnérable à l'oxydation.

Il est donc intéressant d'obtenir une huile naturellement riche en PUFAs, dont la composition est la plus proche possible des substances liposolubles de l'organisme producteur avec un minimum de contaminants produits lors des traitements. Or, l'extraction des huiles riches en PUFA rencontre des difficultés particulières à cette forte teneur en PUFA, qui limitent les quantités d'acides gras extraits de la biomasse.

Les procédés d'extraction utilisés actuellement font intervenir l'ajout de sodium (WO 2011/153246), de solvant (US 2014/350222) et/ou des températures élevées durant plusieurs heures (WO 2015/095694) qui peuvent réduire la qualité de l'huile obtenue et du co-produit (biomasse délipidée). En effet, la température est un facteur induisant la formation d'acides gras *trans* à partir des acides gras *cis.* Or la réglementation impose une teneur maximale inférieure à 1% d'acides gras *trans* dans les huiles alimentaires. Les acides gras des microorganismes producteurs de PUFA sont naturellement en conformation *cis,* toute formation d'acides gras *trans* réduit donc la qualité de l'huile. La température peut aussi provoquer la formation de 2-MCPD, 3-MCPD et de glycidol, composés toxiques dont la teneur est réglementée (glycidol) ou en cours de réglementation (2- et 3-MCPD). Il est important de chercher à optimiser la qualité de l'huile enfin d'en avoir le moins possible, en particulier pour l'utilisation des huiles dans l'alimentation infantile. Dans l'art antérieur, US 2015/176042 divulgue un procédé d'extraction d'huile à partir de micro-organismes impliquant une température élevée et la présence d'un sel.

L'ajout de sodium lors de l'extraction, sous forme de sulfate de sodium ou bien de chlorure de sodium, est un procédé connu (WO 2011/153246). Malheureusement, le sodium se retrouve dans la biomasse délipidée. Cela réduit l'intérêt de cette biomasse, par ailleurs riche en protéines, pour l'alimentation animale notamment. De plus, l'efficacité d'un procédé avec ajout de sodium et des températures inférieure ou égales à 70°C est limitée quant au rendement d'extraction à partir d'une biomasse à plus de 60% de PUFA.

L'invention répond à cette demande avec un nouveau procédé d'extraction des matières grasses à forte teneur en PUFA, qui améliore les rendements d'extraction de ces huiles, et en particulier de l'extraction des PUFA de la biomasse qui les contient, tout en préservant la qualité de l'huile.

### EXPOSE DE L'INVENTION

L'invention concerne un nouveau procédé d'extraction d'une huile riche en PUFA à partir d'une biomasse d'organismes producteurs comprenant ladite huile, le procédé comprenant les étapes (a) de lyse cellulaire sur une suspension de la biomasse d'organisme producteur d'huile et (b) de séparation mécanique de l'huile de la biomasse lysée et récupération de l'huile brute, le procédé se caractérisant en ce que la lyse cellulaire comprend deux parties :
(a1) une première partie mise en œuvre à une première température, puis
(a2) une deuxième partie de poursuite de la lyse à une deuxième température inférieure d'au moins 10°C à la première température,
étant entendu que les étapes (a1) et (a2) sont effectuées sans modification substantielle du milieu de suspension dans lequel la lyse cellulaire a eu lieu.

Le procédé selon l'invention peut être mis en œuvre pour tout type d'organisme producteur d'une huile à forte teneur en PUFA, qu'il s'agisse d'un animal, en particulier un animal marin, de plantes oléagineuses ou de microorganismes.

Le procédé selon l'invention est particulièrement adapté pour l'extraction d'huiles microbiennes à forte teneur en PUFA. Il ne fait pas intervenir l'ajout de solvants pour l'extraction de l'huile.

Aussi décrite mais non comprise dans l'invention telle que revendiquée, est une huile riche en PUFA obtenue par le procédé, qu'elle soit brute ou raffinée ou encore l'huile diluée comprenant l'huile extraite avec le procédé selon l'invention qui est mélangée avec une autre huile.

Aussi décrite mais non comprise dans l'invention telle que revendiquée, est une composition pharmaceutique, cosmétique ou alimentaire qui comprend une huile obtenue par le procédé selon l'invention, qu'elle soit brute, raffinée ou diluée.

Aussi décrite mais non comprise dans l'invention telle que revendiquée, est l'utilisation d'une huile obtenue par le procédé selon l'invention, brute, raffinée ou diluée, pour l'alimentation humaine ou animale, en particulier pour l'alimentation des nouveaux nés, des enfants, ou des femmes enceintes ou allaitantes.

Aussi décrite mais non comprise dans l'invention telle que revendiquée, est une composition, en particulier une composition neutraceutique ou un aliment, qui comprend de l'huile obtenue par le procédé selon l'invention, qu'elle soit brute, raffinée ou diluée.

### DESCRIPTION DETAILLEE DE L'INVENTION

Les PUFA sont bien connus de l'homme du métier, notamment pour leur utilisation comme additifs alimentaires ou pour la préparation d'aliments fonctionnels, notamment pour des laits infantiles ou à destination des femmes enceintes ou allaitantes. On citera en particulier l'acide docosahexaénoïque (DHA, C22:6n3), le DPA (acide docosapentaénoïque, C22:5n6), l'acide arachidonique (ARA, C20:4n6) ou l'acide eicosapentaénoïque (EPA, C20:5n3). Selon les organismes producteurs considérés, les huiles comprennent majoritairement du DHA, de l'EPA ou de l'ARA, ou encore des mélanges de plusieurs PUFA.

Les organismes producteurs d'ARA sont bien connus. On citera en particulier les champignons filamenteux du genre *Mortierella alpina.* Les organismes producteurs d'EPA sont également bien connus. On citera en particulier *Nannochloropsis gaditana .* Les organismes producteurs de DHA sont aussi bien connus. On citera notamment les protistes des genres *Aurantochytrium* ou *Schizochytrium.* Les plantes sont également de bon producteurs de PUFA, en particulier en niveau des graines : L'huile de lin (Linum usitatissimum L.) peut contenir plus de 50% d'acide alpha-linolénique (ALA) ; le colza (*Brassica napus*) est également un producteur d'ALA très connu. Les plantes ne produisent pas d'acides gras à très longues chaines (supérieur à C18). Cependant, récemment, des plantes transgéniques de *Camelina sativa* ont été modifiées pour produire du DHA (Mansour *et al.,* 2014) ou d'EPA (Ruiz-Lopez *et al.* 2014). Les graines des plantes oléagineuse contiennent typiquement entre 20% (soja) et 70% (noix de coco déhydratée) de matière grasse (Rosenthal et al., 1996).

Le procédé selon l'invention est particulièrement adapté pour l'extraction d'huiles riches en PUFA à partir de microorganismes producteurs d'huiles. Ces microorganismes sont particulièrement choisis parmi les champignons filamenteux et les protistes.

Les souches de microorganismes qui permettent d'obtenir de telles huiles sont des souches industrielles, c'est à dire selon l'invention, des souches dont la teneur en matière grasse représente au moins 45 % de la matière sèche, préférentiellement au moins environ 50 % de la matière sèche, et qui ont une capacité de croissance à une densité cellulaire d'au moins 50 g/L, préférentiellement d'au moins 70 g/L, plus préférentiellement d'au moins 100 g/L.

L'homme du métier connaît bien les souches industrielles de microorganismes productrices de PUFA principalement parmi les traustochytrides, les dinophycées, les diatomées, les eustigmatophycées, notamment les microorganismes des genres *Crypthecodinium, Schizochytrium, Traustochytrium* ou *Aurantiochytrium* pour la production de DHA.

On citera en particulier *Crypthecodinium cohnii* M64245, *Crypthecodinium cohnii* FJ821501, *Crypthecodinium cohnii, Crypthecodinium cohnii* CCAP 1104/3 (WO2016030631), *Crypthecodinium cohnii* CCAP 1104/5 or *Crypthecodinium cohnii* CCAP 1104/4, *Aurantiochytrium limacinum* AB022107 *; Aurantiochytrium limacinum* HM042909 ; *Aurantiochytrium limacinum* JN986842 *; Aurantiochytrium limacinum* SL1101 JN986842 ; *Aurantiochytrium mangrovei ; Aurantiochytrium mangrovei* DQ323157 ; *Aurantiochytrium mangrovei* DQ356659 *; Aurantiochytrium mangrovei* DQ367049 *; Aurantiochytrium mangrovei* CCAP 4062/2 ; *Aurantiochytrium mangrovei* CCAP 4062/3 ; *Aurantiochytrium mangrovei* CCAP 4062/4 ; *Aurantiochytrium mangrovei* CCAP 4062/5 ; *Aurantiochytrium mangrovei* CCAP 4062/6 ; *Aurantiochytrium mangrovei* CCAP 4062/1 *; Aurantiochytrium sp.* AB052555 *; Aurantiochytrium sp.* AB073308 ; *Aurantiochytrium sp.* ATCC PRA276 DQ836628 ; *Aurantiochytrium sp.* BL10 FJ821477 ; *Aurantiochytrium sp.* LY 2012 PKU Mn5 JX847361 ; *Aurantiochytrium sp.* LY2012 JX847370 *; Aurantiochytrium sp.* N1-27 *; Aurantiochytrium sp.* SD116 *; Aurantiochytrium sp.* SEK209 AB290574 ; *Aurantiochytrium sp.* SEK217 AB290572 *; Aurantiochytrium sp.* SEK 218 AB290573 *; Aurantiochytrium sp. 18W-13a ; Botryochytrium radiatum ; Botryochytrium radiatum* Raghukumar 16 ; *Botryochytrium radiatum* SEK353 ; *Botryochytrium sp. ; Botryochytrium sp.* BUTRBC 143 ; *Botryochytrium sp.* Raghukumar 29 ; *Oblongichytrium minutum ; Oblongichytrium multirudimentalis ; Oblongichytrium sp. ; Oblongichytrium sp.* SEK347 *; Parieticytrium sarkarianum ; Parieticytrium sarkarianum* SEK351 *; Parieticytrium sarkarianum* SEK364 *; Parieticytrium sp. ; Parieticytrium sp.* F3-1 ; *Parieticytrium sp.* H1-14 *; Parieticytrium sp.* NBRC102984 ; *Phytophthora infestans ; Schizochytrium aggregatum* DQ323159 *; Schizochytrium aggregatum* DQ356661 ; *Schizochytrium aggregatum ; Schizochytrium limacinum ; Schizochytrium limacinum* OUC166 HM042907 ; *Schizochytrium mangrovei ; Schizochytrium mangrovei* FB1 ; *Schizochytrium mangrovei* FB3 *; Schizochytrium mangrovei* FB5 ; *Schizochytrium minutum* ; *Schizochytrium* sp. ATCC20888 DQ367050 *; Schizochytrium sp.* KGS2 KC297137 ; *Schizochytrium sp.* SKA10 JQ248009 *; Schizochytrium sp.* ATCC 20111 ; *Schizochytrium sp.* ATCC 20888 (WO1991007498); *Schizochytrium sp.* ATCC 20889 *; Schizochytrium sp.* ATCC 26185 ; *Schizochytrium sp.* BR2.1.2 *; Schizochytrium sp.* BUCAAA 032 *; Schizochytrium sp.* BUCAAA 093 *; Schizochytrium sp.* BUCACD 152 ; *Schizochytrium sp.* BUCARA 021 *; Schizochytrium* sp. BUCHAO 113 ; *Schizochytrium sp.* BURABQ 13 ; *Schizochytrium sp.* BURARM 802 ; *Schizochytrium sp.* CCAP 4087/3 (WO2017012931) *; Schizochytrium sp.* CCAP 4087/1 ; *Schizochytrium sp.* CCAP 4087/4 ; *Schizochytrium sp.* CCAP 4087/5 *;Schizochytrium sp.* FJU-512 *; Schizochytrium sp.* KH105 ; *Schizochytrium sp.* KK17-3 *; Schizochytrium sp.* KR-5 *; Schizochytrium sp.* PJ10.4 ; *Schizochytrium sp.* SEK 210 ; *Schizochytrium sp.* SEK 345 ; *Schizochytrium sp.* SEK 346 *; Schizochytrium sp.* SR21 ; *Schizochytrium sp.* TIO01 ; *Sicyoidochytrium minutum* SEK354 ; *Sicyoidochytrium minutum* NBRC 102975*Sicyoidochytrium minutum* NBRC 102979 ; *Thraustochytriidae sp.* BURABG162 DQ100295 ; *Thraustochytriidae sp.* CG9 ; *Thraustochytriidae sp.* LY2012 JX847378 ; *Thraustochytriidae sp.* MBIC11093 AB183664 ; *Thraustochytriidae sp.* NIOS1 AY705769 ; *Thraustochytriidae sp.* #32 DQ323161 ; *Thraustochytriidae sp.* #32 DQ356663 ; *Thraustochytriidae sp.* RT49 DQ323167 ; *Thraustochytriidae sp.* RT49 DQ356669 ; *Thraustochytriidae sp.* RT49 ; *Thraustochytriidae sp.* Thel2 DQ323162 ; *Thraustochytriidae sp.* Thel2 ; *Thraustochytrium aggregatum ; Thraustochytrium aggregatum* DQ356662 ; *Thraustochytrium aureum ; Thraustochytrium aureum* DQ356666 ; *Thraustochytrium gaertnerium ; Thraustochytrium kinnei ; Thraustochytrium kinnei* DQ323165 ; *Thraustochytrium motivum ; Thraustochytrium multirudimentale ; Thraustochytrium pachydermum ; Thraustochytrium roseum ; Thraustochytrium sp.* 13A4.1 *; Thraustochytrium* sp. ATCC 26185 ; *Thraustochytrium sp.* BL13 ; *Thraustochytrium sp.* BL14 ; *Thraustochytrium sp.* BL2 ; *Thraustochytrium sp.* BL3 ; *Thraustochytrium sp.* BL4 ; *Thraustochytrium sp.* BL5 ; *Thraustochytrium sp.* BL6 ; *Thraustochytrium sp.* BL7 ; *Thraustochytrium sp.* BL8 ; *Thraustochytrium sp.* BL9 ; *Thraustochytrium sp.* BP3.2.2 ; *Thraustochytrium sp.* BP3.3.3 ; *Thraustochytrium sp.* caudivorum ; *Thraustochytrium sp.* CHN-1 *; Thraustochytrium sp.* FJN-10 ; *Thraustochytrium sp.* HK1 *; Thraustochytrium sp.* HK10 ; *Thraustochytrium sp.* HK5 ; *Thraustochytrium sp.* HK8 ; *Thraustochytrium sp.* HK8a ; *Thraustochytrium sp.* KK17-3 ; *Thraustochytrium sp.* KL1 ; *Thraustochytrium sp.* KL2 ; *Thraustochytrium sp.* KL2a ; *Thraustochytrium sp.* ONC-T18 ; *Thraustochytrium sp.* PJA10.2 ; *Thraustochytrium sp.* TR1.4 ; *Thraustochytrium sp.* TRR2 ; *Thraustochytrium striatum ; Thraustochytrium striatum* ATCC24473 (WO2017131188); *Thraustochytrium striatum* DQ323163 ; *Thraustochytrium striatum* DQ356665 ; *Thraustochytrium visurgense ; Ulkenia amoeboidea* SEK 214 ; *Ulkenia profunda ; Ulkenia profunda* BUTRBG 111 *; Ulkenia sp ; Ulkenia sp.* ATCC 28207 (WO1998003671) ; *Ulkenia visurgensis ; Ulkenia visurgensis* BURAAA 141 *; Ulkenia visurgensis* ATCC 28208 ; Thraustochytrides déposées à l'ATCC sous les numéros d'accession PTA-9695, PTA9696, PTA-9697 et PTA-9698 (US 2010-239533);.

Pour la production d'huiles riches en EPA on citera *Phaeodactylum tricornutum* (Pt1) Bohlin Strain 8.6 CCMP2561 (WO2015008160) ; Nitzschia brevirostris (CCAP 1052/21 ; WO2013136025) ; Nitzschia laevis (UTEX 2047 ; WO2008004900) ; Pythium irregulare (US9074160) ; *Nannochloropsis limnetica* (WO2016059262) ; *Nannochloropsis salina; Nannochloropsis avicula ; Nannochloropsis acceptata ; Nannochloropsis oculata* CCAP849/1 ; *Nannochloropsis pseudotenelloides ; Nannochloropsis gaditana ; Nannochloropsis sp.* CCAP211/46 ; *Nannochloropsis saprophila ; Chlorella protothecoides* CCAP211/17 (WO201115041); *Chlorella ellipsoidea; Chlorella minutissima ; Chlorella zofinienesi ; Chlorella luteoviridis* CCAP211/3 ; *Chlorella kessleri ; Chlorella sorokiniana* CCAP211/8K ; *Chlorella fiusca var. vacuolata ; Chlorella sp. ; Chlorella emersonii ; Monodus subterraneus.*

Pour la production d'huiles riches en ARA on citera *Mortierella elongata* IFO8570, *Mortierella exigua* IF08571, *Mortierella hygrophila* IF05941, *Mortierella alpina* IF08568, ATCC16266, ATCC32221, ATCC42430, CBS219.35, CBS224.37, CBS250.53, CBS343.66, CBS527.72, CBS529.72, CBS608.70, and CBS754.68 (WO 2015/095696).

Le procédé selon l'invention est particulier adapté pour extraire les PUFA des organismes qui comprennent des huiles riches en PUFA, en particulier à des teneurs comprenant plus de 50% de PUFA par rapport à la masse totale de matière grasse. Il trouve ses meilleures applications pour extraire les PUFA des microorganismes qui produisent des huiles qui comprennent plus de 60% de PUFA par rapport à la masse totale de matière grasse, avantageusement au moins 62% de PUFA, plus avantageusement au moins 65% de PUFA, de préférence plus de 67%, plus préférentiellement au moins 70%, encore plus préférentiellement 75% de PUFA par rapport à la masse totale de matière grasse.

Toutefois, les bénéfices observés de manière significative sur les fortes teneurs en PUFA se retrouve également pour extraire les PUFA de microorganismes qui produisent des huiles moins riches, mais avec des quantités qui restent significatives de PUFA, de 40% à 50% de PUFA par rapport à la masse totale de matière grasse.

Selon un mode préféré de réalisation de l'invention, le PUFA à extraire est du DHA, ou un mélange DHA+DPA, les microorganismes employés étant des microorganismes producteur d'huiles riches en DHA ou en DHA+DPA.

On citera plus particulièrement *Aurantiochytrium mangrovei CCAP 4062*/*2 ; Aurantiochytrium mangrovei CCAP 4062*/*3 ; Aurantiochytrium mangrovei CCAP 4062*/*4 ; Aurantiochytrium mangrovei CCAP 4062*/*5 ; Aurantiochytrium mangrovei CCAP 4062*/*6 ; Aurantiochytrium mangrovei CCAP 4062*/*1 ; Schizochytrium sp. CCAP 4087*/*3 ; Schizochytrium sp. CCAP 4087*/*1 ; Schizochytrium sp. CCAP 4087*/*4 ; Schizochytrium sp. CCAP 4087*/*5 ;* ou les *Thraustochytrides* déposées à l'ATCC sous les numéros d'accession PTA-9695, PTA9696, PTA-9697 et PTA-9698. Le procédé selon l'invention pourra être mis en œuvre avec des microorganismes qui produisent des huiles particulièrement riches en DHA, en particulier les souches *Aurantiochytrium mangrovei* CCAP4062/7 et CCAP4062/8 et *Schizochytrium sp.* CCAP4087/7.

La biomasse d'organismes producteurs peut subir un premier traitement pour permettre cette lyse cellulaire. Par exemple, pour des graines de plantes oléagineuses, la biomasse pourra subir un ensemble de traitements connus pour séparer les grains des enveloppes, puis un premier broyage et une mise en suspension du broyat avant de mettre en œuvre la lyse.

Pour une biomasse de microorganismes issus d'une culture de microorganismes, la biomasse peut être directement issue du mout de fermentation. Elle pourra être lavée pour éliminer certains solubles (en particulier par filtration pour éliminer le milieu de fermentation et lavage à l'eau) et remise en suspension pour l'étape (a) de lyse. La biomasse de microorganisme peut aussi être une biomasse séchée ou lyophilisée qui a été conservée et qui est remise en suspension préalablement à l'étape de lyse. Ces prétraitements de la biomasse microbienne pour faciliter la mise en œuvre de l'étape (a) de lyse sont bien connus de l'homme du métier.

L'étape (a) de lyse cellulaire est mise en œuvre sur une suspension de biomasse. Cette suspension a de préférence une teneur en matière sèche de 5 à 20% en masse, généralement de 8 à 10% en masse.

La lyse cellulaire est faite par lyse enzymatique ou mécanique. La température de la première partie de lyse est de préférence d'au moins 50°C tout en restant inférieure à des températures qui viendraient dégrader la composition des huiles en plus de favoriser la lyse cellulaire, c'est à dire des températures inférieures à 95°C, voire inférieures à 90°C, de préférence inférieures à 80°C, encore plus préférentiellement d'au plus 70 °C.

On connaît les enzymes susceptibles d'être employées, notamment décrites dans WO2015/095688, WO2011/153246, US6750048 et WO2015/095694, en particulier des proteases ou des cellulases telles que les enzymes commercialisées par la société Novozyme sous les appellations Alcalase 2,5 L, Alcalase 2,4 L, Alcalase 3,0 T, Novozym 37071, Flavourzyme 1000 L, Novozym FM 2,4 L, Protamex, Viscozyme. Les conditions de mise en œuvre sont celles préconisées par le fournisseur, la température étant celle préconisée pour une activité optimale des enzymes, d'au moins 50°C et jusqu'à 70 °C, de préférence d'environ 65°C. Avantageusement la lyse enzymatique est mise en œuvre sous une atmosphère pauvre en oxygène. Généralement, la concentration en oxygène est inférieure à 1% en masse.

Les méthodes de lyse mécaniques sont également bien connues, notamment par broyeur à billes, mélangeur-disperseur, homogénéisateur haute pression, broyeur à broches ou broyeur à impact, ultra-sons, champs électrique pulsés. Comme dispositifs pour la mise en œuvre de ces méthodes de lyse mécanique, on citera notamment (nom du constructeur entre parenthèses) pour le broyeur à billes : Discus-1000 (Netzsch); ECM-AP60 (WAB) ; pour l'homogénéisateur haute pression: Ariete (GEA) ; pour le mélangeur-disperseur : 700-X (Silverson), pour le broyeur à broche : Contraplex (Hosakawa); pour le broyeur à impact : Condux (Netzsch).

La première partie (a1) de la lyse est mise en œuvre dans les conditions usuelles préconisées de l'état de la technique pour une lyse cellulaire, notamment en terme de durée de la lyse enzymatique ou les cycles de broyages.

L'étape (a2) de poursuite de la lyse permet de compléter cette dernière en modifiant les conditions de mise en œuvre sans avoir à extraire la biomasse lysée au préalable. La modification des conditions de mise en œuvre est une modification de température, mais ne comprend pas de modifications substantielle du milieu de suspension comprenant la biomasse partiellement lysée, comme l'ajout de sel, ou d'acide ou de base. Une modification substantielle du milieu de suspension préalable ou concomitante à la baisse de température n'est pas a priori une « poursuite de la lyse » selon l'invention. De telles modifications substantielles du milieu de suspension après la lyse sont décrites dans les demandes US 2011/295028 et US 2015/176042 qui ne permettent pas une extraction efficace des huiles à forte teneur en PUFA.

Avantageusement, la lyse enzymatique consiste essentiellement en ces deux parties (a1) et (a2) qui se distinguent essentiellement par une baisse de température lorsque l'on passe à la deuxième partie (a2) de la lyse.

La température de lyse dans cette deuxième partie est inférieure d'au moins 10°C à celle de la première partie. De préférence, la température de la seconde partie de lyse est inférieure ou égale à 40°C, avantageusement allant de 5°C à 40°C, plus avantageusement allant de 10°C à 35°C, voire de 15°C à 30°C, de préférence de 20°C à 30°C. Cette deuxième partie (a2) de lyse à température inférieure, ou fin de lyse, est avantageusement mise en œuvre pendant au moins 30', pouvant aller jusqu'à 30 h. L'état de l'art nous enseigne de maintenir la température au-dessus de 40°C, voire d'augmenter la température au-delà de 80°C pour améliorer l'extraction durant ou bien après l'étape de lyse, ceci que la biomasse de départ soit des microorganismes (WO2018011275) ou bien des graines oléagineuses (Rosenthal *et al.,* 1996). Les inventeurs ont trouvé de manière surprenante que l'inverse (baisse de température) donne un meilleur résultat, plus particulièrement pour les huiles à forte teneur en PUFA. La température de l'étape (a2) n'est pas augmentée en fin de lyse avant l'étape (b) de séparation.

La séparation mécanique (b) d'une huile à partir d'une biomasse lysée est également bien connue de l'homme du métier, comme une séparation gravitaire, notamment par centrifugation comme décrite dans la demande de brevet WO 01/53512. On peut aussi employer une séparation continue, en particulier par séparateur centrifuge à assiettes. De tels séparateurs sont connus pour extraire en continu des huiles de milieux complexes comprenant des résidus solides et de l'eau, tels que décrits dans la demande de brevet WO 2010/096002, notamment commercialisés par les sociétés Alfa Laval, Flottweg ou GEA Westfalia, notamment. Cette étape de séparation continue est préférée dans le procédé employé pour obtenir l'huile selon l'invention.

Selon la teneur en PUFA de l'organisme producteur, les huiles brutes obtenues par le procédé selon l'invention peuvent avoir de très fortes teneurs en PUFA, en particulier en DHA, de plus de 60% de PUFA par rapport à la masse totale de matière grasse, avantageusement au moins 62% de PUFA, plus avantageusement au moins 65% de PUFA, de préférence plus de 67%, plus préférentiellement au moins 70%, encore plus préférentiellement 75% de PUFA par rapport à la masse totale de matière grasse extraite.

Les huiles obtenues par le procédé selon l'invention sont essentiellement sous la forme de triglycérides. Les triglycérides représentent avantageusement au moins 80% de la masse totale de matière grasse, de manière plus avantageuse au moins 90%, de manière encore plus avantageuse au moins 93% de la masse totale de matière grasse. La teneur en triglycérides est par exemple analysée par chromatographie sur couche mince (Jouet et al., 2003).

Généralement, l'extraction de l'huile à partir de la biomasse avec le procédé selon l'invention permet d'obtenir une légère augmentation de la teneur en PUFA, en particulier en DHA et en DPA, favorisant l'extraction de ces PUFA par rapport aux acides gras saturés de plus bas poids moléculaire. Toutefois, cette concentration ne modifie pas de manière substantielle les propriétés intrinsèques de l'huile contenue dans la biomasse, notamment la teneur en triglycérides. Dans tous les cas, l'huile selon l'invention est une huile qui n'a pas subi de modifications substantielles de sa teneur en acides gras par l'ajout de PUFA, par exemple sous forme d'esters, par concentration et/ou par l'élimination d'acides gras saturés comme l'acide palmitique.

L'huile obtenue est généralement une huile appelée huile brute, qui peut être employée telle quelle ou faire l'objet d'un raffinage, notamment pour faciliter sa conservation, en évitant qu'elle ne rancisse, ou pour modifier sa couleur de manière à la rendre plus acceptable pour un consommateur. Ces étapes de raffinage sont bien connues de l'homme du métier, notamment de dégommage, de neutralisation des acides gras libres, de décoloration et de désodorisation. Elles permettent d'éliminer (tout ou bien en partie) les phospholipides, les pigments, les volatiles et les acides gras libres. De fait, ces méthodes ne viennent pas modifier substantiellement la teneur relative en acides gras, saturés ou insaturés, ni la teneur en triglycérides de l'huile raffinée obtenue par rapport à l'huile brute.

L'invention concerne donc aussi un procédé d'extraction d'une huile riche en PUFA tel que défini plus haut, qui comprend également une étape (c) de rafinage.

Certains procédés de l'état de la technique présentent une étape dite de « winterisation » mise en œuvre sur les huiles brutes ou raffinées, notamment pour éliminer les acides gras saturés, avec pour effet d'augmenter la teneur en PUFA (WO 02/10322). Les huiles extraites selon l'invention, brutes ou raffinées, ne nécessitent a priori pas de « winterization » pour être exploitées. Toutefois, l'homme du métier pourra choisir d'ajouter une telle étape de « winteriation » s'il y trouve un quelconque avantage commercial pour son produit final.

Selon les microorganismes producteurs d'huiles riches en PUFA employés, notamment des microorganismes producteurs de DHA, l'huile obtenue par le procédé selon l'invention peut contenir plus 10 mg de caroténoïdes natifs par kg d'huile, voire plus de 30 mg/kg, préférentiellement plus de 40 mg/kg, encore plus préférentiellement plus de 60 mg/kg, voire au moins 65 mg/kg. Les caroténoïdes présents sont majoritairement de l'astaxanthine et des béta-carotènes. L'huile contient plus de 20 mg/kg d'astaxanthine, voire plus de 30 mg/kg, plus préférentiellement plus de 40 mg/kg. La cantaxanthine est également présente mais en quantité moindre. D'autres caroténoïdes comme la lutéine et la zéaxanthine peuvent être présents mais ils sont à la limite de détection de la méthode utilisée. Le terme « caroténoïdes natifs » signifie que les caroténoïdes n'ont pas été ajoutés, ils proviennent de la même biomasse que l'huile et sont extraits de cette biomasse en même temps que l'huile. Ils sont produits par la souche dans des conditions de fermentation en hétérotrophie, sans stimulus particulier. Ces caroténoïdes natifs sont donc présents tout au long du procédé, protégeant les acides gras, en particulier le DHA, contre l'oxydation. Le procédé de raffinage peut éliminer les pigments, donc l'huile raffinée pourra contenir moins, voire plus du tout, de caroténoïdes.

Les huiles brutes ou raffinées peuvent aussi être diluées pour leur usage ultérieur.

L'invention concerne donc aussi un procédé d'extraction d'une huile riche en PUFA tel que défini précédemment, qui comprend en outre une étape (d) de dilution de l'huile brute obtenue à l'étape (b) de séparation mécanique ou de l'huile raffinée obtenue à l'étape (c) de raffinage.

Les huiles employées pour diluer l'huile riche en PUFA obtenue par le procédé selon l'invention sont généralement et de préférence des huiles végétales adaptées à une consommation alimentaire humaine ou animale. On citera en particulier les huiles de tournesol, de colza, de soja, de noix, de sésame, de chanvre, de noisette, d'argan, d'olive, de lin, ou de toute autre huile adaptée à un usage alimentaire. L'huile ajoutée peut aussi être une huile comprenant d'autres PUFA, en particulier du DHA, de l'ARA et/ou de l'EPA, en particulier d'autres huiles d'origine microbienne ou encore des huiles de poisson.

La présente demande décrit aussi une composition qui comprend une huile riche en PUFA obtenue par le procédé selon l'invention, qu'elle soit brute, raffinée ou diluée.

Une composition telle que décrite peut comprendre un ou plusieurs excipients. Un excipient est un composant, ou mélange de composants, qui est utilisé dans la présente invention pour donner des caractéristiques souhaitables à la composition pour sa conservation et son usage, y compris des aliments et des compositions pharmaceutiques, cosmétiques et industrielles. Un excipient peut être décrit comme un excipient "pharmaceutiquement acceptable" lorsqu'il est ajouté à une composition pharmaceutique dont les propriétés sont connues de la pharmacopée pour être employés au contact des tissus humains et animaux sans toxicité excessive, irritation, réaction allergique ou autres complications. Différents excipients peuvent être utilisés comme une base organique ou minérale, un acide organique ou minéral, un tampon de pH, un stabilisant, un antioxydant, un agent d'adhésion, un agent de séparation, un agent de revêtement, un composant de phase extérieure, un composant à libération contrôlée, un agent tensioactif, un humectant, une charge, un émollient ou des combinaisons de ceux-ci.

Selon leur destination, les compositions selon l'invention sont en particulier des compositions pharmaceutiques, cosmétiques, neutraceutiques ou des aliments.

Les aliments sont destinés tant aux humains qu'aux animaux et comprennent des compositions solides, pâteuses ou liquides. On citera en particulier les aliments courants, les produits liquides, y compris laits, boissons, boissons thérapeutiques et boissons nutritionnelles, les aliments fonctionnels, les suppléments, les neutraceutiques, les préparations pour nourrissons, y compris les préparations pour nourrissons prématurés, les aliments pour femmes enceintes ou allaitantes, les aliments pour adultes, les aliments gériatriques et aliments pour animaux.

L'huile riche en PUFA obtenue par le procédé selon l'invention, qu'elle soit brute ou raffinée ou la biomasse qui la contient peut être utilisée directement comme ou ajouté en tant qu'additif dans une huile, une pâte à tartiner, un autre ingrédient gras, une boisson, une sauce à base de soya ou à base de soja, des produits laitiers (lait, yaourt, fromage, crème glacée), des produits de boulangerie, des produits nutritionnels, par exemple sous forme de complément nutritionnel (sous forme de gélule ou de comprimé), des suppléments vitaminiques, des compléments alimentaires, des poudres à diluer pour boissons, comme des boissons énergétiques ou des poudres de laits pour des formulations infantiles, des produits alimentaires en poudre fini ou semi-fini, etc., selon les usages connus de l'homme du métier.

Les aliments pour animaux sont également connus de l'homme du métier. Ils sont en particulier destinés aux animaux d'élevage, comme les vaches, cochons, poulets, moutons, chèvres ou dans la pisciculture pour les crustacés ou les poissons d'élevage.

Les compositions pharmaceutiques comprenant une huile riche en PUFA sont également connues de l'homme du métier, l'huile étant employée seule ou en combinaison avec d'autres médicaments.

L'huile riche en PUFA obtenue par le procédé selon l'invention, brute ou raffinée ou la biomasse qui la contient, peut être formulée sous la forme de compositions unidoses, notamment sous forme de comprimés, de gélules, de capsules, de poudres, de granulés, adaptés à une administration per os.

La présente demande décrit également l'utilisation d'une huile riche en PUFA obtenue par le procédé selon l'invention, brute, raffinée ou diluée, pour l'alimentation humaine ou animale, en particulier pour l'alimentation des nouveaux nés, des enfants, ou des femmes enceintes ou allaitantes.

De tels usages sont bien connus de l'homme du métier, notamment décrits dans la demande de brevet WO 2010/107415 et sur le site Web de la société DSM (https://www.dsm.com/markets/foodandbeverages/en_US/products/nutritional-lipids/life-dha.html)

### EXEMPLES

### EXEMPLE 1 : Cultures en fermenteur de souches à haute teneur en DHA

Les cultures sont réalisées dans des fermenteurs (bioréacteurs) de 1 à 5 L utiles avec automates dédiés et supervision par station informatique. Elles sont effectuées à partir de deux souches d'*Aurantiochytrium mangrovei* et avec deux protocoles de cultures différents. Le système est régulé à pH 5 via l'ajout de base (NH4OH pour exemple b1 et b2 et avec NaOH pour exemple a) avec ajustement du pH réalisé sur tout la durée de la culture, et fournissant une alimentation d'azote (dans le cadre des exemples b1 et b2). La température de culture a été fixée à 30°C puis 22°C et enfin 18°C sur la fin de la culture.

La souche CCAP4062/7 est utilisée pour l'exemple a et b1 tandis que la souche FCCB1897 est utilisée pour l'exemple b2.

La composition des milieux de culture est donnée dans le Tableau 1.

**Tableau 1**

| | **a** | **b1 et b2** | |
|---|---|---|---|
| CaCl2, 2H2O | 0,55 | 0,55 | g/L |
| MgSO4, 7H2O | 4-8 | 4-8 | g/L |
| H3BO3 | 0,00875-0,175 | 0,00875-0,0175 | g/L |
| K2SO4 | 2,08 | 0,00 | g/L |
| KH2PO4 | 4,00 | 4,00 | g/L |
| Na4EDTA, 2H2O | 0,12 | 0,12 | g/L |
| FeSO4, 7H2O | 0,04 | 0,04 | g/L |
| (NH4)2SO4 | 9,00 | 1,00-2,00 | g/L |
| MnCl2, 4H2O | 0,0108 | 0,0108 | g/L |
| ZnSO4, 7H2O | 0,0108 | 0,0108 | g/L |
| CoCl2, 6H2O | 0,000108 | 0,000108 | g/L |
| Na2MoO4, 2H2O | 0,000108 | 0,000108 | g/L |
| Na2SeO3 | 1,73E-07 | 1,73E-07 | g/L |
| CuSO4, 5H2O | 0,0072 | 0,0072 | g/L |
| NiSO4, 6H2O | 0-0,0056 | 0-0,0056 | g/L |
| Thiamine | 0,0320 | 0,0320 | g/L |
| Vitamine B12 | 0,0005 | 0,0005 | g/L |
| Panthotenate | 0,0108 | 0,0108 | g/L |
| Antimousse Biospumex 153K | 0,40 | 0,40 | mL/L |
| Glucose, 1 H2O | 55,00 | 55,00 | g/L |

Des ajouts de glucose sous forme d'une solution d'enrichissement sont faits avec un ratio molaire de carbone : azote : phosphore (CNP) de 533 : 11 : 1 (exemple a) soit avec une solution composée uniquement de glucose (exemples b1 et b2).

### Suivi des cultures :

La concentration en biomasse totale est suivie par mesure de la masse sèche (filtration sur filtre GF/F, Whatman, puis séchage en étuve, à 105°C, pendant 24 h minimum avant pesée). Les analyses en acides gras sont réalisées selon une méthode adaptée de la norme ISO 12966-2 pour la biomasse, et selon la Pharmacopée Européenne 9.0 (2.4.29.) pour les huiles.

Les profils en acides gras des biomasses obtenues avec les conditions a, b1 et b2 sont donnés dans le Tableau 2, en pourcentage par rapport aux acides gras totaux. AGS : acides gras saturés.

**Tableau 2**

| | **a** | **b1** | **b2** |
|---|---|---|---|
| C10 :0 | 0,0 | 0,0 | 0,0 |
| C11 :0 | 0,0 | 0,0 | 0,0 |
| C12 :0 | 0,0 | 0,0 | 0,0 |
| C13 :0 | 0,0 | 0,0 | 0,0 |
| C14 :0 | 0,8 | 1,2 | 0,3 |
| C14 :1 | 0,0 | 0,0 | 0,0 |
| C15 :0 | 0,0 | 0,1 | 1,7 |
| C15 :1 | 0,0 | 0,0 | 0,0 |
| C16 :0 | 13,6 | 19,6 | 10,9 |
| C16 :1 | 0,1 | 0,2 | 0,2 |
| C16 :2 | 0,0 | 0,0 | 0,0 |
| C16 :3 | 0,0 | 0,0 | 0,0 |
| C16 :4 | 0,0 | 0,0 | 0,0 |
| C17 :0 | 0,0 | 0,0 | 0,0 |
| C17 :1 | 0,0 | 0,0 | 0,3 |
| C18 :0 | 0,5 | 0,6 | 0,6 |
| C18 :1 | 0,2 | 0,3 | 0,4 |
| C18 :2 | 0,0 | 0,0 | 0,0 |
| C18 :3n3 | 0,2 | 0,1 | 0,2 |
| C18 :3n6 | 0,1 | 0,1 | 0,1 |
| C18 :4n3 | 0,3 | 0,3 | 0,3 |
| C20 :0 | 0,1 | 0,1 | 0,1 |
| C20 :4n6 (ARA) | 0,1 | 0,0 | 0,1 |
| C20 :5n3 (EPA) | 0,4 | 0,6 | 0,6 |
| C21 :0 | 0,0 | 0,1 | 0,0 |
| C22 :0 | 0,1 | 0,0 | 0,0 |
| C22 :5n3 (DPAn3) | 0,2 | 0,0 | 0,2 |
| C22 :5n6 (DPAn6) | 12,9 | 9,6 | 12,7 |
| C22 :6n3 (DHA) | 66,6 | 62,5 | 70,1 |
| **DHA+DPA** | **79,7** | **72** | **83** |
| **AGS** | **15,1** | **22** | **11,4** |
| **DHA/DPA** | **5,2** | **6,5** | **5,5** |
| **DHA/AGS** | **4,4** | **2,9** | **6,2** |
| **(DHA+DPA)/AGS** | **5,3** | **3,3** | **7,3** |

### EXEMPLE 2 : Culture en conditions industrielles de souches à haute teneur en DHA

Les souches *d'Aurantiochytrium mangrovei à* haute teneur en DHA produisent une biomasse de composition similaire en acides gras lorsque qu'elles sont cultivées en fermenteurs de taille industrielle, comme des cuves de 10 m3 (exemple d) ou bien de 180 m3 (exemple e), selon des conditions similaires, avec le milieu de culture b et des ajouts de glucose sous forme d'une solution d'enrichissement sont faits avec un ratio molaire de carbone : azote : phosphore (CNP) de 533 : 0,4 : 1 .

Les profils en acides gras de la biomasse pour les exemples d et e sont donnés dans le Tableau 3, en pourcentage par rapport aux acides gras totaux.

**Tableau 3**

| | **10m3** | **180m3** |
|---|---|---|
| C10 :0 | 0,0 | 0,0 |
| C11 :0 | 0,0 | 0,0 |
| C12 :0 | 0,0 | 0,0 |
| C13 :0 | 0,0 | 0,0 |
| C14 :0 | 0,7 | 0,5 |
| C14 :1 | 0,0 | 0,0 |
| C15 :0 | 0,0 | 0,1 |
| C15 :1 | 0,0 | 0,0 |
| C16 :0 | 18,5 | 13,9 |
| C16 :1 | 0,1 | 0,1 |
| C16 :2 | 0,0 | 0,0 |
| C16 :3 | 0,0 | 0,0 |
| C16 :4 | 0,0 | 0,0 |
| C17 :0 | 0,0 | 0,0 |
| C17 :1 | 0,0 | 0,3 |
| C18 :0 | 0,8 | 0,6 |
| C18 :1n9 (c+t) | 0,0 | 0,0 |
| C18 :2n6 (c+t) | 0,0 | 0,0 |
| C18 :3n3 | 0,3 | 0,2 |
| C18 :3n6 | 0,1 | 0,1 |
| C18 :4n3 | 0,4 | 0,3 |
| C20 :0 | 0,1 | 0,1 |
| C20 :4n6 (ARA) | 0,2 | 0,1 |
| C20 :5n3 (EPA) | 0,9 | 0,5 |
| C21 :0 | 0,0 | 0,0 |
| C22 :0 | 0,0 | 0,0 |
| C22 :5n3 (DPAn3) | 0,2 | 0,2 |
| C22 :5n6 (DPAn6) | 11,5 | 13,7 |
| C22 :6n3 (DHA) | 65,5 | 68,3 |
| **Acides gras (% MS)** | **44,0** | **60,0** |
| **DHA+DPA** | **77,0** | **82,2** |
| **AGS** | **20,1** | **15,2** |
| **DHA/DPA** | **5,6** | **4,9** |
| **DHA/AGS** | **3,3** | **4,5** |
| **(DHA+DPA)/AGS** | **3,8** | **5,4** |

### EXEMPLE 3 : extraction de l'huile à partir de la biomasse des souches à haute teneur en DHA : comparaison de rendement avec différents procédés

L'huile est extraite à partir d'une même biomasse, produite selon les conditions de l'exemple 2 et contenant plus de 60% de DHA (tableau 4).

**Tableau 4**

| Composition en acides gras de la biomasse | |
|---|---|
| C14 :0 | 0,9 |
| C16 :0 | 18,1 |
| C18 :0 | 0,6 |
| C18 :1n-9 (c+t) | 0,0 |
| C18 :2n-6 (c+t) | 0,0 |
| C18 :3n-3 | 0,1 |
| C20 :4n-6 | 0,1 |
| C20 :5n-6 (EPA) | 0,7 |
| C22 :5n-6 (DPA) | 9,8 |
| C22 :6n-3 (DHA) | 63,9 |
| Acides gras (% MS) | 50,7 |
| **DHA+DPA** | 73,7 |
| **AGS** | 20,6 |
| **DHA/DPA** | 6,52 |
| **DHA/AGS** | 3,1 |
| **(DHA+DPA)/AGS** | 3,6 |

L'extraction est réalisée selon différents procédés connus de l'homme du métier, impliquant notamment une lyse chimique (WO2015/095688A1) ou une lyse enzymatique et ajout de sulfate de Sodium (WO2011/153246 ) ou des changements de pH (WO2015/095694). Les rendements d'extraction de la matière grasse par rapport aux quantités présentes dans la biomasse sont indiqués dans le tableau 5. Les essais 1, 2 et 3 sont réalisés selon l'enseignement de l'état de la technique. L'essai 4 est réalisé selon l'invention.

**Tableau 5 : rendement d'extraction de l'huile par rapport à la matière grasse présente dans la biomasse, selon les conditions des différents essais d'extraction. MS : matière sèche, MF : matière fraiche.**

| conditions | Essai 1 | Essai 2 | Essai 3 | Essai 4 |
|---|---|---|---|---|
| Lyse enzymatique | Oui : Alcalase (1,2% MS) 2H à 60°C pH 7,3 | Oui : Alcalase (1,2% MS) 2H à 60°C pH 7,3 | non | Oui : Alcalase (1,2% MS) 7H à 65°C pH 8 |
| Hydrolyse acide | non | non | Oui : H₂SO₄ (2,5% MF) 5H40 à 70°C pH 0,5 | non |
| Ajout d'acide ou de soude | NaOH 5H à 70°C pH 12, puis H₂SO₄ 15H à 70°C pH 10,5 | Na₂SO₄ (10% MF) 25H à 21°C pH 7,3 | NaOH 10%, 16H à 70°C pH 7,7 | Non, 17H à **30°C** pH 8 |
| Changement avant centrifugation | H₂SO₄, pH 7 | 50°C 1H avant centrifugation | non | non |
| séparation | centrifugation | centrifugation | centrifugation | centrifugation |
| Rendement % MG | 22% | <1% | <1% | 53% |

### EXEMPLE 4 : extraction de l'huile à partir de la biomasse des souches à haute teneur en DHA

L'extraction de l'huile à partir de de la biomasse produite dans l'exemple 2 est réalisée en suivant la séquence :
(a) lyse cellulaire par voie enzymatique (ex : avec l'Alcalase 2,5 L ou Alcalase 2,4 L ou Novozym 37071 de Novozymes) pendant 4h à une température de 65°C,
(b) poursuite de la lyse en abaissant la température comprise entre 5 et 40°C, pendant une durée comprise entre 30 minutes et 30h,
(c) séparation mécanique de l'huile par séparateur centrifuge à assiettes.

Le rendement d'extraction est de 60% de lipides extraits de la biomasse.

**Tableau 6 : profil lipidique de l'huile extraite**

| | **10 m3** | **180 m3** |
|---|---|---|
| C10 :0 | 0,0 | 0,0 |
| C11 :0 | 0,0 | 0,0 |
| C12 :0 | 0,0 | 0,0 |
| C13 :0 | 0,0 | 0,0 |
| C14 :0 | 0,3 | 0,0 |
| C14 :1 | 0,0 | 0,0 |
| C15 :0 | 0,0 | 0,0 |
| C15 :1 | 0,0 | 0,0 |
| C16 :0 | 15,4 | 8,3 |
| C16 :1 | 0,0 | 0,0 |
| C16 :2 | 0,0 | 0,0 |
| C16 :3 | 0,0 | 0,0 |
| C16 :4 | 0,0 | 0,0 |
| C17 :0 | 0,0 | 0,0 |
| C17 :1 | 0,0 | 0,0 |
| C18 :0 | 0,4 | 0,4 |
| C18 :1n9 (c+t) | 0,0 | 0,0 |
| C18 :2n6 (c+t) | 0,0 | 0,0 |
| C18 :3n3 | 0,0 | 0,0 |
| C18 :3n6 | 0,0 | 0,0 |
| C18 :4n3 | 0,2 | 0,0 |
| C20 :0 | 0,0 | 0,0 |
| C20 :4n6 (ARA) | 0,0 | 0,0 |
| C20 :5n3 (EPA) | 0,5 | 0,2 |
| C21 :0 | 0,0 | 0,0 |
| C22 :0 | 0,0 | 0,0 |
| C22 :5n3 (DPAn3) | 0,0 | 0,0 |
| C22 :5n6 (DPAn6) | 11,6 | 15,4 |
| C22 :6n3 (DHA) | 71,5 | 75,5 |
| **DHA+DPA** | **83,1** | **90,9** |
| **AGS** | **16,1** | **8,7** |
| **DHA/DPA** | **6,2** | **4,9** |
| **DHA/SFA** | **4,4** | **8,7** |
| **(DHA+DPA)/AGS** | **5,2** | **10,4** |

### REFERENCES

- EP 0 223 960 ; EP 1 001 034
- WO 1994/008467 ; WO 1997/037032 ; WO 2001/054510 ; WO 02/10322; WO 03/049832 ; WO 2010/107415 ; WO2011/153246 ; WO 2012/035262 ; WO 2013/136025 ; WO 2013/136028 ; WO 2014/146098 ; WO 2015/004402 ; WO 2015/004403 ; WO2015/095688 ; WO2015/095694 ; WO 2015/150716 ; WO 2016/030631
- US 6,750,048, US 2011/295028, US 2015/176042, US 2014/350222
- Fedorova-Dahms I. & al., Safety evaluation of DHA-rich algal oil from Schizochytrium sp, Food and Chemical Toxicology, 2011, 49, 3310-3318
- Folch J, et al., A simple method for the isolation and purification of total lipides from animal tissues. J Biol Chem. 1957 May; 226(1):497-509
- Hamilton M. & al., Heterotrophic Production of Omega-3 Long-Chain Polyunsaturated Fatty Acids by Trophically Converted Marine Diatom Phaeodactylum tricornum, Marine Drugs, 2016, 14, 53
- Omega-3 long chain fatty acid "bioavailability": a review of evidence and methodological considerations.
- Ghasemifard S, Turchini GM, Sinclair AJ. Prog Lipid Res. 2014 Oct;56:92-108. doi: 10.1016/j.plipres.2014.09.001. Epub 2014 Sep 16. Review.
- Wakako TSUZUKI, Study of the Formation of trans Fatty Acids in Model Oils (triacylglycerols) and Edible Oils during the Heating Process , JARQ 46 (3), 215 - 220 (2012)
- Kinuko Miyazaki* and Kazuo Koyama, An Improved Enzymatic Indirect Method for Simultaneous Determinations of 3-MCPD Esters and Glycidyl Esters in Fish Oils , J. Oleo Sci. 66, (10) 1085-1093 (2017)
- Jouhet J., Marechal E., Bligny R., Joyard J., Block M. A. (2003). Transient increase of phosphatidylcholine in plant cells in response to phosphate deprivation. FEBS Lett. 544 63-68.
- Maged P. Mansour,1,* Pushkar Shrestha,2 Srinivas Belide,2 James R. Petrie,2 Peter D. Nichols,1 and Surinder P. Singh2 (2014). Characterization of Oilseed Lipids from "DHA-Producing Camelina sativa": A New Transformed Land Plant Containing Long-Chain Omega-3 Oils. Nutrients. 2014 Feb; 6(2): 776-789.
- Ruiz-Lopez N, Haslam RP, Napier JA, Sayanova O (2014) Successful high-level accumulation of fish oil omega-3 long chain polyunsaturated fatty acids in a transgenic oilseed crop. Plant J. 77(2):198-208
- Rosenthal A, Pyle DL, and Niranjan K (1996) Aqueous and enzymatic processes for edible oil extraction. Enzyme and Microbial Technology 19:402-420.

## Revendications

1. Procédé d'extraction d'une huile riche en acides gras polyinsaturés (PUFA) à partir d'une biomasse d'organismes producteurs comprenant ladite huile, le procédé comprenant les étapes (a) de lyse cellulaire sur une suspension de la biomasse d'organisme producteur d'huile et (b) de séparation mécanique de l'huile de la biomasse lysée et récupération de l'huile brute, **caractérisé en ce que** la lyse cellulaire (a) comprend deux parties :
(a1) une première partie mise en œuvre à une première température, puis
(a2) une deuxième partie de poursuite de la lyse à une deuxième température inférieure d'au moins 10°C à la première température,
étant entendu que les étapes (a1) et (a2) sont effectuées sans modification substantielle du milieu de suspension dans lequel la lyse cellulaire a lieu.

2. Procédé selon la revendication 1, **caractérisé en ce que** la biomasse d'organismes producteurs est une biomasse de microorganismes producteurs d'acides gras polyinsaturés (PUFA).

3. Procédé selon la revendication 2, **caractérisé en ce que** la biomasse est une suspension de microorganismes.

4. Procédé selon l'une des revendications 1 à 3, **caractérisé en ce que** l'huile produite par les organismes producteurs comprend plus de 50% de PUFA.

5. Procédé selon l'une des revendications 1 à 4, **caractérisé en ce que** les PUFA sont choisis parmi l'acide docosahexaénoïque (DHA, C22:6n3), l'acide docosapentaénoïque (DPA, C22:5n6), l'acide arachidonique (ARA, C20:4n6), l'acide eicosapentaénoïque (EPA, C20:5n3) et leurs mélanges.

6. Procédé selon l'une des revendications 1 à 5, **caractérisé en ce que** l'étape (a) de lyse est une lyse mécanique.

7. Procédé selon l'une des revendications 1à 5, **caractérisé en ce que** l'étape (a) de lyse est une lyse enzymatique.

8. Procédé selon l'une des revendications 1 à 5, **caractérisé en ce que** la température de la première partie de lyse (a1) est d'au moins 50°C et inférieure à 95°C.

9. Procédé selon la revendication 8, **caractérisé en ce que** la température de la première partie de lyse (a1) est d'au plus 70 °C.

10. Procédé selon l'une des revendications 1 à 9, **caractérisé en ce que** la température de la seconde partie de lyse (a2) est inférieure ou égale à 40°C.

11. Procédé selon la revendication 10, **caractérisé en ce que** la température de la seconde partie de lyse (a2) va de 20°C à 30°C.

12. Procédé selon l'une des revendications 1 à 11, **caractérisé en ce que** la séparation mécanique (b) d'une huile à partir d'une biomasse lysée est une séparation continue par séparateur centrifuge à assiettes.

13. Procédé selon l'une des revendications 1 à 12, **caractérisé en ce qu'**il comprend en outre une étape (c) de raffinage de l'huile brute obtenue à l'étape (b).

14. Procédé selon l'une des revendications 1 à 13, **caractérisé en ce qu'**il comprend en outre une étape (d) de dilution de l'huile brute obtenue à l'étape (b) ou de l'huile raffinée obtenue à l'étape (c).

## Patentansprüche

1. Verfahren zur Extraktion eines an mehrfach ungesättigten Fettsäuren (PUFA) reichen Öls aus einer Biomasse von produzierenden Organismen, die das Öl umfassen, wobei das Verfahren die Schritte (a) der Zelllyse auf einer Suspension der Biomasse des ölproduzierenden Organismus und
(b) der mechanischen Trennung des Öls von der lysierten Biomasse und Rückgewinnung des Rohöls umfasst, **dadurch gekennzeichnet, dass** die Zelllyse (a) zwei Teile umfasst:
(a1) einen ersten Teil, der bei einer ersten Temperatur umgesetzt wird, dann
(a2) einen zweiten Teil der Fortsetzung der Lyse bei einer zweiten Temperatur, die mindestens 10 °C unter der ersten Temperatur liegt,
wobei die Schritte (a1) und (a2) ohne wesentliche Änderung des Suspensionsmediums, in dem die Zelllyse stattfindet, durchgeführt werden.

2. Verfahren nach Anspruch 1, **dadurch gekennzeichnet, dass** die Biomasse von produzierenden Organismen eine Biomasse von mehrfach ungesättigten Fettsäuren produzierenden Mikroorganismen (PUFA) ist.

3. Verfahren nach Anspruch 2, **dadurch gekennzeichnet, dass** die Biomasse eine Suspension von Mikroorganismen ist.

4. Verfahren nach einem der Ansprüche 1 bis 3, **dadurch gekennzeichnet, dass** das von den produzierenden Organismen hergestellte Öl mehr als 50 % PUFA umfasst.

5. Verfahren nach einem der Ansprüche 1 bis 4, **dadurch gekennzeichnet, dass** die PUFAs unter Docosahexaensäure (DHA, C22:6n3), Docosapentaensäure (DPA), C22:5n6), Arachidonsäure (ARA, C20:4n6), Eicosapentaensäure (EPA, C20:5n3) und deren Mischungen ausgewählt sind.

6. Verfahren nach einem der Ansprüche 1 bis 5, **dadurch gekennzeichnet, dass** der Schritt (a) der Lyse eine mechanische Lyse ist.

7. Verfahren nach einem der Ansprüche 1 bis 5, **dadurch gekennzeichnet, dass** der Schritt (a) der Lyse eine enzymatische Lyse ist.

8. Verfahren nach einem der Ansprüche 1 bis 5, **dadurch gekennzeichnet, dass** die Temperatur des ersten Lyseteils (a1) mindestens 50 °C und weniger als 95 °C beträgt.

9. Verfahren nach Anspruch 8, **dadurch gekennzeichnet, dass** die Temperatur des ersten Lyseteils (a1) höchstens 70 °C beträgt.

10. Verfahren nach einem der Ansprüche 1 bis 9, **dadurch gekennzeichnet, dass** die Temperatur des zweiten Lyseteils (a2) kleiner oder gleich 40 °C ist.

11. Verfahren nach Anspruch 10, **dadurch gekennzeichnet, dass** die Temperatur des zweiten Lyseteils (a2) von 20 °C bis 30 °C beträgt.

12. Verfahren nach einem der Ansprüche 1 bis 11, **dadurch gekennzeichnet, dass** die mechanische Trennung (b) eines Öls von einer lysierten Biomasse eine kontinuierliche Trennung mittels Tellerspaltseparator ist.

13. Verfahren nach einem der Ansprüche 1 bis 12, **dadurch gekennzeichnet, dass** es ferner einen Schritt (c) der Raffinierung des in Schritt (b) erhaltenen Rohöls umfasst.

14. Verfahren nach einem der Ansprüche 1 bis 13, **dadurch gekennzeichnet, dass** es ferner einen Schritt (d) der Verdünnung des in Schritt (b) gewonnenen Rohöls oder des in Schritt (c) gewonnenen raffinierten Öls umfasst.

## Claims

1. A process for extracting PUFA-rich oil from biomass of producing organisms comprising said oil, the process comprising the steps (a) of cell lysis on a suspension of the biomass of oil-producing organism and
(b) of mechanical separation of the oil from the lysed biomass and recovery of the crude oil, **characterized in that** the cell lysis (a) comprises two parts:
(a1) a first part implemented at a first temperature, then
(a2) a second part of the continuation of the lysis at a second temperature lower than at least 10°C from the first temperature, it being understood that steps (a1) and (a2) are carried out without substantial modification of the suspension medium in which the cellular lysis takes place.

2. A process according to claim 1, **characterized in that** the biomass of producing organisms is a biomass of PUFA-producing microorganisms.

3. A process according to claim 2, **characterized in that** the biomass is a suspension of microorganisms.

4. A process according to any one of claims 1 to 3, **characterized in that** the oil produced by the producing organisms comprises more than 50% PUFA.

5. A process according to any one of claims 1 to 4, **characterized in that** the PUFAs are selected from docosahexaenoic acid (DHA, C22:6n3), docosapentaenoic acid (DPA, C22:5n6), arachidonic acid (ARA, C20:4n6), eicosapentaenoic acid (EPA, C20:5n3) and mixtures thereof.

6. A process according to any one of claims 1 to 5, **characterized in that** the lysis step (a) is a mechanical lysis.

7. A process according to any one of claims 1 to 5, **characterized in that** the lysis step (a) is an enzymatic lysis.

8. A process according to any one of claims 1 to 5, **characterized in that** the temperature of the first lysis step (a1) is at least 50°C and less than 95°C.

9. A process according to claim 8, **characterized in that** the temperature of the first lysis part (a1) is at most 70°C.

10. A process according to one of claims 1 to 9, **characterized in that** the temperature of the second lysis part (a2) is less than or equal to 40°C.

11. A process according to claim 10, **characterized in that** the temperature of the second lysis part (a2) ranges from 20°C to 30°C.

12. A process according to one of claims 1 to 11, **characterized in that** the mechanical separation (b) of an oil from a lysed biomass is a continuous separation by a disc-stack centrifugal separator.

13. Process according to one of claims 1 to 12, **characterized in that** it further comprises a step (c) of refining the crude oil obtained at step (b).

14. Process according to one of claims 1 to 13, **characterized in that** it further comprises a step (d) of diluting the crude oil obtained at step (b) or the refined oil obtained at step (c).
